Europäisches Patentamt

European Patent Office

Office européen des brevets

(11) Publication number: **0 292 000 B1**

(12) **EUROPEAN PATENT SPECIFICATION**

(45) Date of publication of patent specification: **16.09.92**  (51) Int. Cl.5: **A61K 37/00**, A61K 39/05, A61K 39/21

(21) Application number: **88108127.7**

(22) Date of filing: **20.05.88**

(54) **Treatment of viral infection.**

(30) Priority: **22.05.87 JP 126794/87**

(43) Date of publication of application:
**23.11.88 Bulletin 88/47**

(45) Publication of the grant of the patent:
**16.09.92 Bulletin 92/38**

(84) Designated Contracting States:
**AT BE CH DE ES FR GB GR IT LI LU NL SE**

(56) References cited:
**EP-A- 0 017 507**
**EP-A- 0 240 346**
**EP-A- 0 247 873**
**US-A- 4 709 017**

(73) Proprietor: **SUMITOMO CHEMICAL COMPANY, LIMITED**
**Kitahama 4-chome 5-33**
**Chuo-ku Osaka 541(JP)**

(72) Inventor: **Ueda, Shigeharu**
**5-A10-408, Fujishirodai 3-chome**
**Suita-shi Osaka-fu(JP)**
Inventor: **Ikuta, Kazuyoshi**
**18-1-117, Yamadahigashi 3-chome**
**Suita-shi Osaka-fu(JP)**
Inventor: **Kato, Shiro**
**23-7, Fujishirodai 4-chome**
**Suita-shi Osaka-fu(JP)**
Inventor: **Uchida, Tsuyoshi**
**24-B-701, Kamishinden 1-chome**
**Toyonaka-shi Osaka-fu(JP)**

(74) Representative: **Vossius & Partner**
**Siebertstrasse 4 P.O. Box 86 07 67**
**W-8000 München 86(DE)**

EP 0 292 000 B1

## Description

The present invention relates to a therapeutic composition for the treatment of AIDS and to a method for its preparation.

AIDS, which is caused by infection with human immunodeficiency virus (HIV), is producing serious social problems in the world, and various therapeutic agents for treatment of AIDS are under development, of which typical examples are interferon, interleukin, azidothymidine (AZT), etc. However, their therapeutic effects are still not sufficiently reliable.

Diphtheria toxin is a single chain protein having a molecular weight of about 60,000. Said toxin exerts strong cytotoxicity against human beings, and its lethal dose for adult is from about 10 to 20 $\mu$g. Restricted decomposition of said toxin by treatment with trypsin in the presence of a reducing agent under mild conditions gives fragment A having a molecular weight of about 20,000 and fragment B having a molecular weight of about 40,000 (Collier, R.J. et al. (1971), I. Thiol-dependent dissociation of a fraction of toxin into enzymatically active and inactive fragments, J.Biol.Chem., 246:1496-1503; Gill, D.M. et al. (1971), Observations on the structure of diphtheria toxin, J.Biol.Chem., 246:1485-1491). Said fragment A inhibits protein synthesis in cells causing killing of the cells, while fragment B performs the role of introducing fragment A into cells by binding onto the surfaces of the cells. Thus, the cytotoxicity of diphtheria toxin is produced by a combination of fragments A and B, and each of fragments A and B does not exert any material cytotoxicity by itself (Uchida, T. et al. (1972), Reconstitution of diphtheria toxin from two nontoxic cross-reacting mutant toxins, Science 175:901-903). In other words, fragment A alone, does not exert any material cytotoxicity outside cells, and its cytotoxicity is less than $10^{-4}$ of that of diphtheria toxin. When, however, fragment A is introduced into a cell by any artificial means, thesole molecule is sufficient to kill the cell (Yamaizumi, M. et al. (1978), One molecule of diphtheria toxin fragment A introduced into a cell can kill the cell, Cell, 15:245-250).

Inhibition of the protein synthesis by fragment A is made through its catalyzation of the following reaction (1) by inactivation of the elongation factor 2 (EF-2), which is a proteinous factor essential for elongation of a peptide chain. In the elongation process, peptidyl t-RNA is transferred from the A site to the P site (Honjo, T. et al. (1968), Diphtheria-toxin-dependent adenosine diphosphate ribosylation of aminoacyl transferase II and inhibition of protein synthesis, J.Biol.Chem. 243:2553-3555):

$$\text{EF-2} + \text{NAD}^+ \rightarrow \text{ADP-Ribose-EF-2} + \text{nicotinamide} + \text{H}^+ \qquad (1)$$

According to the reaction (1), EF-2 binds covalently to ADP-Ribose and is inactivated, whereby protein synthesis is inhibited. The above reaction is highly specific, and no substrate other than EF-2 has been found.

When liposomes containing fragment A are injected into the brain of an animal with viral encephalitis, said fragment is selectively introduced into enveloped virus-infected cells so that the protein synthesis in such cells is completely inhibited resulting in destruction of said infected cells. Simultaneously, the proliferation of virus as well as the extension of the infection is prevented. Accordingly, said liposomes are useful for treatment of viral encephalitis such as subacute sclerosing panencephalitis, measles encephalitis and herpetic encephalitis (Japanese Patent Publication (Unexamined) No. 252425/85).

In addition to the fragment A of diphtheria toxin, a number of other toxins are known which specifically inhibit protein synthesis of a cell. The C-terminal active portion of Pseudomonas exotoxin and fragment A derived from castor oil plant seeds are examples of such toxins.

The present inventors have found that liposomes containing a toxin specifically inhibiting the protein synthesis in a cell, especially fragment A of diphtheria toxin, can attack and selectively kill HIV-infected cells, and that said liposomes are quite useful as a therapeutic agent for the treatment of AIDS. This invention is based on the above finding.

Accordingly, the present invention provides the use of a toxin which specifically inhibits cellular protein synthesis for the manufacture of a pharmaceutical composition for the treatment of acquired immunodeficiency syndrome (AIDS). The pharmaceutical composition comprises preferably a liposomal preparation containing the toxin.

In the present invention, said fragment A of diphtheria toxin is preferably used as the toxin specifically inhibiting cellular protein synthesis, but such known toxins as the C-terminal active portion of Pseudomonas exotoxin or fragment A derived from castor oil plant weeds can also be used.

This invention will be hereinafter explained taking fragment A of diphtheria toxin (hereinafter referred to as "fragment A") as a representative example. The scope of the invention is not limited to the case wherein liposomes containig fragment A of diphtheria toxin are used but covers cases wherein liposomes or other suitable pharmaceutical carriers containing other toxins specifically inhibiting cellular protein synthesis are used.

As stated above, fragment A of diphtheria toxin is obtained from diphtheria toxin by a conventional procedure. For instance, diphtheria toxin as produced by Corynebacterium diphtheriae is treated with trypsin in the presence of a reducing agent, and the restrictively decomposed product is purified to recover fragment A. Alternatively, cultivation of a fragment A-producing mutant C7 ($\beta$ 22) of Corynebacterium diphtheriae gives fragment A alone, i.e. not contaminated with fragment B (Uchida, et al., (1979), Reconstitution of lipid vesicles associated with HVJ (Sendai virus) spikes; Purification and some properties of vesicles containing nontoxic fragment A of diphtheria toxin, J.Cell Biol., 80:10-20). Said mutant C7 ($\beta$ 22) is available from the Division of Cellular Biology, Institute for Molecular and Cellular Biology, Osaka University, located at Suita, Osaka, Japan.

The liposomes themselves can be readily prepared by known methods. In a typical example, the liposomes may be prepared from a phospholipid, preferably lecithin, and cholesterol. Phosphatidylserine or phosphatidylethanolamine may be added thereto. The mixing proportion of the phospholipid and cholesterol is not critical insofar as the resulting mixture should maintain the form of liposomes at low temperatures,for instance, about 0 to 10°C, and it may be usually from about 1 : 5 to 1 : 8 by weight. The weight proportion of phosphatidylserine and/or phosphatidylethanolamine, if added, may be about 1/2 - 1/3 (preferably about 1/2) of the used cholesterol.

For the preparation of the fragment A-containing liposomes, the phospholipid and cholesterol may be admixed with fragment A in a small quantity of 0.01 M phosphate buffer containing 0.5 % by weight of a non-ionic surfactant (e.g. octylglycoside, "Nonidet P40") and 0.25 - 0.3 M sucrose, followed by removal of the surfactant by a conventional sepration procedure such as dialysis, gel filtration or adsorption. The weight of fragment A to be admixed may usually be from about 1 to 1/5 of that of the phospholipid. Fragment A not contained in the liposomes can be removed by gel filtration using a gel filtration agent, preferably "Bio-Gel"® A50M (manufactured by Bio Rad).

The prepared fragment A-containing liposomes can be used for the treatment or prevention of diseases the caused by HIV. For such purpose, the fragment A-containing liposomes may be administered intravenously to each patient, for example, at a dose of 1 to 5 ml once a week. The dose may be appropriately varied according to the symptoms, the body-weight, etc.

It has been found that AZT effectively prevents HIV-uninfected cells from infection with HIV derived from the infected cells or free HIV remaining after said treatment with the liposomal preparation of the invention. Thus, the combined use of the fragment A-containing liposomes with AZT is particularly effective in the preventive or therapeutic treatment of AIDS.

When HIV-infected cells are cultured in a medium containing the fragment A-containing liposomes, fragment A is introduced into said cells so that all protein syntheses in said cells is inhibited, and as the result, said cells are killed. The proliferation of HIV is thus inhibited, and the extension of the infection is prevented.

It is assumed that the fragment A-containing liposomes cannot reach the lipid bilayer of the plasma membrane in HIV-uninfected cells, and that, in case of HIV-infected or HIV-producing cells, HIV infection and/or production changes the surface structure of the cells so that the fragment A-containing liposomes can reach the lipid bilayer of the cell surface. Presumably, because of this reason, the fragment A-containing liposomes exert their cytotoxicity selectively on HIV-infected or HIV-producing cells.

The anti-HIV antibody which can recognize an env protein in HIV does not affect the cytotoxicity of the fragment A-containing liposomes to HIV-infected cells. This means that HIV-infected or HIV-producing cells in AIDS patients having the HIV antibody or HIV-infected persons can be killed by treatment with the fragment A-containing liposomes.

Since HIV-infected or HIV-producing cells in AIDS patients or HIV-infected persons are mainly present in their blood, it seems to be quite easy to bring the fragment A-containing liposomes into contact with said cells. Thus, the fragment A-containing liposomes of the invention have an excellent preventive or therapeutic effect in the treatment of AIDS. More specifically, the fragment A-containing liposomes of the invention are characteristic in exerting excellent preventive or therapeutic effect in the treatment of AIDS without undesirable effects on uninfected normal cells.

The preparation procedures of the liposomal preparation of the invention will be hereinafter explained in detail taking fragment A of diphtheria toxin as a representative example. For the preparations of liposomes containing other toxins, substantially the same procedures can be used.

Preparation of fragment A of diphtheria toxin:-

The mutant C7 ($\beta$ 22) of <u>Corynebacterium</u> <u>diphtheriae</u> is cultured in a nutrient medium. The cells are removed from the culture medium by centrifugation. Ammonium sulfate is added to the supernatant to precipitate fragment A. The precipitated fragment A is collected, dialyzed to remove ammonium sulfate, adsorbed on DEAE-cellulose and eluted therefrom using NaCl solutions having gradient concentrations, whereby fragment A almost free from any contaminant is obtained. Final purification is made by gel-filtration using G-150. The yield of fragment A as the purified product from about 5 liters of the supernatant is about 20 mg.

Preparation of liposomes containing fragment A of diphtheria toxin:-

A mixture of lecithin and cholesterol (5 : 1 by weight) is added to 0.01 M tris buffer (pH 7.5 ) to make a concentration of 2 mg/ml. Then, fragment A is added to make concentrations of 1 mg/ml to 500 $\mu$g/ml. The resultant mixture is stirred well, and "Nonidet" P40 (NP40) is added thereto to make a concentration of 0.5 % by weight. Instead of NP40, alkylphenol polyoxyethylene ether ("Triton"® X100; manufactured by Rohm & Haas) may be used. On addition of such neutral surfactant, the lipid is solubilized to give a clear solution. Removal of the neutral surfactant from the clear solution results in liposomes containing fragment A. When using NP40, dialysis is effected with a dyalizing tube ("Spectrapore" No. 2; manufactured by Spectrum Medical Industries, Inc.) to remove NP40.

If "Triton"® X 100, is used, stirring is carried out in the presence of "Biobeads"® SM2 (manufactured by Bio Rad) so that "Triton"® X 100 is adsorbed on "Biobeads"®. In order to remove fragment A not contained in the liposomes, the product is treated with "Bio-Gel"® A50m (manufactured by Bio Rad). The thus obtained suspension of the purified fragment A-containing liposomes contains fragment A in amounts of 2 to 5 $\mu$g/ml.

This invention will be hereinafter explained in detail by way of examples. However, these examples should not be construed to limit the the invention thereto and are merely illustrative.

Example 1

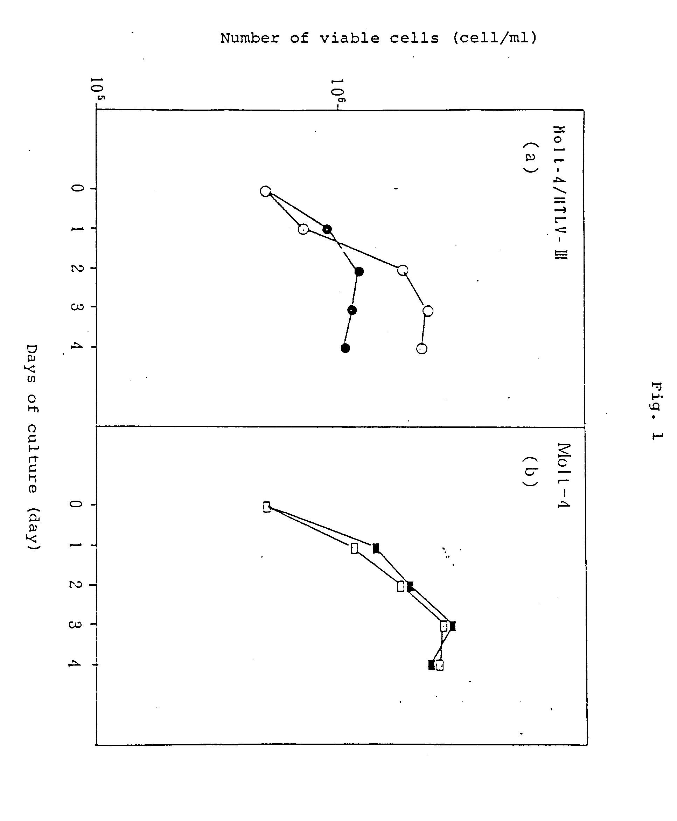

A test tube containing HIV-producing human cells (Molt-4/HTLV-III) (5 x $10^6$ cells) was subjected to centrifugation, and the supernatant was removed. A medium (RPMI-1640 containing 10 % fetal calf serum) (1 ml) was added thereto to make a cell suspension. The suspension was divided into two 0.5 ml portions, of which one was combined with 0.35 ml of phosphate buffered saline (PBS) alone, and the other was combined with 0.35 ml of a suspension of the liposomes containing fragment A of diphtheria toxin (fragment A content, 1.40 $\mu$g). Both of them were kept in a $CO_2$-incubator (37°C, 5 % $CO_2$) for 1.5 hours, and then 5 ml of fresh medium (RPMI-1640 containing 10 % fetal calf serum) was added thereto. The number of cells was counted daily and plotted to give proliferation profiles as shown in Fig.1.

The same procedure as above was repeated using virus-uninfected Molt-4 in place of Molt-4/HTLV-III.

In Fig. 1 wherein the abscissa indicates the day of culture and the ordinate indicates the number of cells, (a) and (b) show respectively the case with Molt-4/HTLV-III wherein -●- is the fragment A-containing liposomes added and -○- is the fragment A-containing liposomes not added and the case with Molt-4 wherein -■- is the fragment A-containing liposomes added and -□- is the fragment A-containing liposomes not added. In case of virus-infected cells (Molt-4/HTLV-III), apparent suppression of cell proliferation was produced by addition of the fragment A-containing liposomes. In case of virus-uninfected cells (Molt-4), the substantially same proliferation profile was obtained irrespective of addition of the fragment A-containing liposomes. The above results indicate that proliferation of virus-infected cells was specifically inhibited by the fragment A-containing liposomes.

Example 2

A mixture of lecithin and cholesterol (5 : 1 by weight) was admixed with fragment A of diphtheria toxin (5 mg). The resultant mixture was solubilized with 0.5 % NP40 in 1 ml 0.01 M phosphate buffer (pH 7.2) containing 0.3 M sucrose. Then, the mixture was dialyzed against the same buffer as above at 0 to 1°C for several days and subjected to gel filtration with "Bio-Gel"® A50m to remove fragment A not contained in liposomes, whereby liposomes containing fragment A of diphtheria toxin were obtained.

HIV-uninfected cells and HIV-producing cells of acute lymphocytic leukemia (ALL) derived cells, Molt-4 and Tall-1, and also HTLV I-infected cells (MT-4) were cultured in media (RPMI-1640 containing 10 % fetal calf serum). Each of the cell cultures (cell concentration, 2.5 x $10^6$ cells/ml) was admixed with a suspension of the fragment A-containing liposomes (concentration of fragment A, 3.33 $\mu$g/ml) (0.19 $OD_{540nm}$) or the

4

same volume of PBS as control. The obtained mixture was incubated while shaking at 37°C for 2 hours, diluted with RPMI-1640 containing 10 % fetal calf serum to adjust the cell concentration to $5 \times 10^5$ cells/ml and incubated at 37°C in a $CO_2$-incubator. The number of cells was counted every 24 hours for two days. In the above procedure, two HIV types were used, i.e. HTLV-III isolated in U.S.A. and LAV isolated in France (Popovic, M. et al., Science, 224, 497 (1987); Levy, J.A. et al., ibid., 225, 840 (1984); Barre-Sinoussi, A. et al., ibid, 220, 868 (1983)).

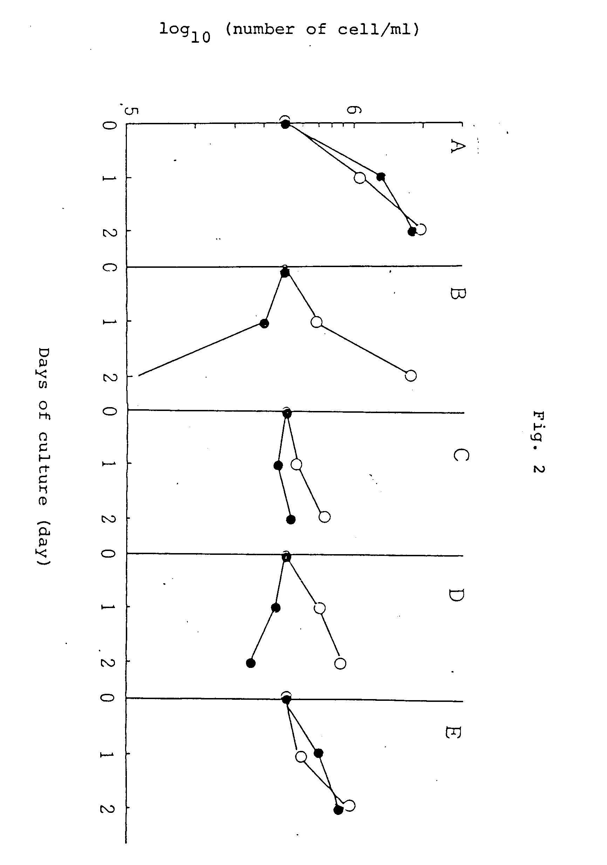

The results are shown in Fig. 2 wherein the abscissa indicates the day of culture and the ordinate indicates the number of cells. A, B, C, D and E show respectively results obtained with HIV-uninfected cells (Molt-4), with HTLV-III-producing cells (Molt-4/HTLV-III), with HIV-uninfected cells (Tall-1), with HIV-producing cells (Tall-1/LAV) and with HTLV-1-infected cells (MT-4), -●- indicates the addition of fragment A-containing liposomes and -O- indicates the addition of PBS only. The fragment A-containing liposomes showed no toxicity against HIV-uninfected cells (Molt-4) and HTLV-1-infected cells (MT-4) (Fig 2, A and E), while they showed slight toxicity against virus-uninfected cells (Tall-1) (Fig. 2, C). In case of HTLV-III-producing cells (Molt-4/HTLV-III), the number of cells decreased to 6.2 % of the control value (treated with PBS) after treatment with the fragment A-containing liposomes (Fig. 2, B). Similarly, in case of HIV-producing cells (Tall-1/LAV), the number of cells decreased to 40.0 % of the control value (Fig. 2, D).

From these results, it is clear that the surface structure of ALL-derived cells become sensitive to the fragment A-containing liposomes by HIV production and that the fragment A-containing liposomes exert a selective cytotoxicity against HIV-producing cells.

### Example 3

MT-4 cells were infected with HIV obtained from a culture of Molt-4/HTLV-III cells and then cultured for 2 days. In the same manner as in Example 2, the resulting cells were treated with the fragment A-containing liposomes. Controls were treated with PBS.

Then, each of the suspensions of cells adjusted to a cell concentration of $5 \times 10^5$ cells/ml was cultured in the presence or absence of AZT (5 $\mu$M) in a medium (RPMI-1640 containing 10 % fetal calf serum) at 37°C for 24 hours. Cell activity was measured by the incorporation of $^3$H-thimidine. Thus, the cells in 200 $\mu$l of each cell suspension were pulse-labeled with 10 $\mu$l of $^3$H-thymidine (40 $\mu$Ci/ml i.e. 1.5 MBq/ml) (manufactured by Amersham, 23 Ci/mmol i.e. 0.85 TBq/mmol) for 2 hours, and $^3$H-thymidine incorporation was determined by counting trichloroacetic acid (TCA)-insoluble radioactivity.

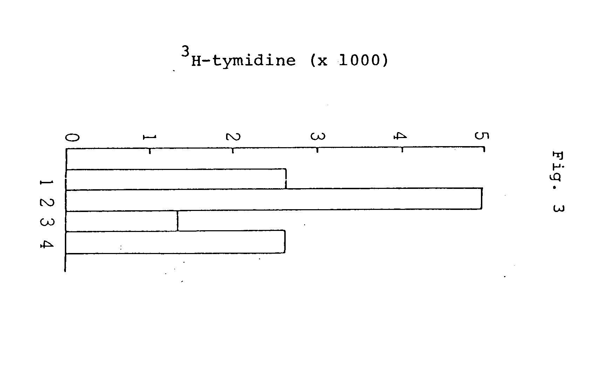

The results are shown in Fig. 3 wherein the ordinate indicates the amount of $^3$H-thymidine incorporated into HIV-infected cells (MT-4), and 1, 2, 3 and 4 respectively correspond to the case wherein treatment was made with the fragment A-containing liposomes but not with AZT, the case wherein treatment was made with the fragment A-containing liposomes and also with AZT, the case wherein treatment was made with PBS but not with AZT and the case wherein treatment was made with PBS and also with AZT.

From Fig. 3, it is understood that $^3$H-thymidine incorporation into cells treated with the fragment A-containing liposomes (1, 2) is greater than that into cells treated with PBS (3). The maximum $^3$H-thymidine incorporation was observed in the cells treated with fragment A-containing liposomes and with AZT. These results suggest that the surface structure of MT-4 cells becomes sensitive to binding of the fragment A-containing liposomes as a result of HIV infection and that AZT inhibits infection of uninfected MT-4 cells with cell-derived or free HIV remained after the treatment with the fragment A-containing liposomes.

### Example 4

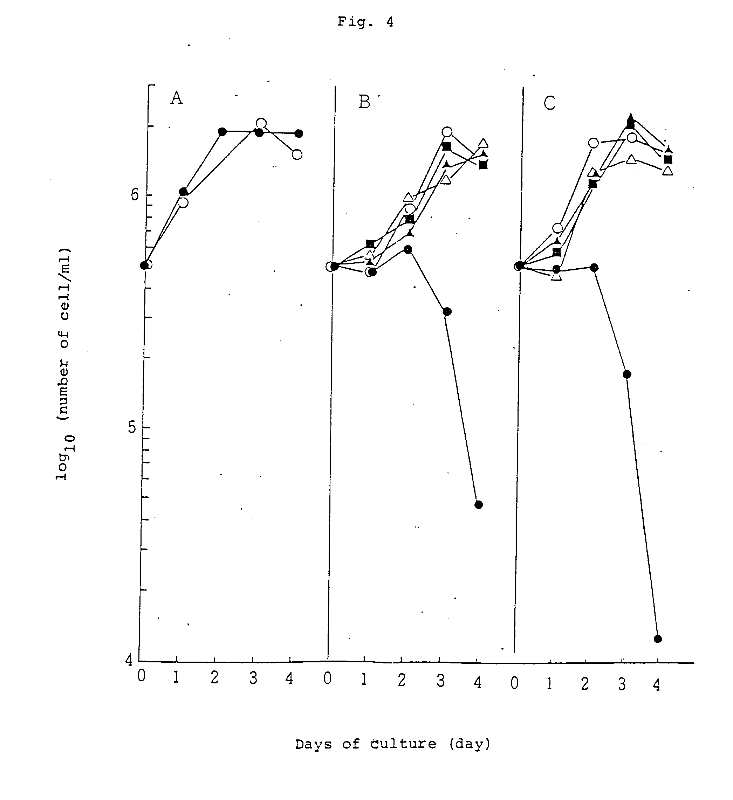

The effect of the anti-HIV antibody on the treatment with the fragment A-containing liposomes was examined, and the results are shown in Fig. 4 wherein the abscissa indicates the day of culture and the ordinate indicates the cell concentration.

Molt-4/HTLV-III cells were treated with a 500-fold dilution of the serum of AIDS virus-infected patients (IF titer: 1:4096) at 37°C for 2 hours. The resulting cells, i.e. antibody-treated Molt-4/HTLV-III cells, and antibody-untreated Molt-4/HILV-III cells were treated with the fragment A-containing liposomes (fragment A concentration, 3.57 $\mu$g/ml) (0.33 $OD_{540\ nm}$) (-●-). As a control, each of said cells was also treated with the liposomes (0.33 $OD_{540\ nm}$) not containing fragment A (-▲-), fragment A alone (concentration, 1.67 $\mu$g/ml) (-■-), a mixture of the liposomes (0.33 $OD_{540\ nm}$) not containing fragment A and fragment A (concentration, 1.67 $\mu$g/ml) (-Δ-) or PBS (-O-). Further, untreated and uninfected cells (Molt-4) were treated with the fragment A-containing liposomes or PBS. The cell numbers were counted every 24 hours for 4 days.

Fig. 4, A, B and C respectively correspond to antibody untreated and HIV-uninfected cells (Molt-4), antibody-untreated and HTLV-III-producing cells (Molt-4/HTLV-III) and antibody-treated and HTLV-III-producing cells (Molt-4/HTLV-III).

As shown in Fig. 4, cytotoxicity in Molt-4/HTLV-III cells was observed only when treated with the fragment A-containing liposomes (Fig. 4, B). The anti HIV-antibody had no influence on the cytotoxicity of the fragment A-containing liposomes (Fig. 4, C). The fragment A-containing liposomes showed no toxic effect on uninfected cells (Fig. 4, A).

Example 5

On the 3rd day after treatment with one of the fragment A-containing liposomes and four kinds of control fluids as used in Example 4, the infectious virus titers of the Molt-4/HTLV-III cell culture medium and the cellular fraction were determined by the immunofluorescence (IF) procedure using a 1000-fold dilution of the serum of AIDS virus-infected patients (IF titer, 1:4096). The cell suspension was fractionated into the cells and the culture medium, and the separated cells were suspended in a culture medium having the same volume as the original culture medium. The $TCID_{50}$/ml value of each specimen was measured using MT-4 cells. MT-4 cells ($1 \times 10^6$ cells/ml; 100 $\mu$l) were placed into each flat well of a microplate and inoculated with the same volume of each of serial 10-fold dilutions of the culture medium or the cell suspension.

After incubation at 37°C for 5 days, the cells were smeared, fixed with cold acetone for 10 minutes and labeled with anti-HIV serum. The Reed and Muench method was used for the determination of the 50 percent end point (Reed, L.J. et al., AM.J.Hyg., 27, 493 (1938)). The results are shown in Table 1.

The infectious virus titer of the culture medium of Molt-4/HTLV-III cells treated with the fragment A-containing liposomes after fractionation of the cells therefrom was reduced to 4.6 % of those which were treated with a mixture of the liposomes not containing fragment A and fragment A. As to the cellular fraction, the HIV infectivity of the HIV-infected cells treated with the fragment A-containing liposomes decreased to 1.0 % of that of the HIV-infected cells treated with a mixture of the liposomes not containing fragment A and fragment A. The HIV infectivity of the culture medium and the cells treated with other control fluids such as the liposomes not containing fragment A, fragment A or PBS were nearly equal to those which were treated with a mixture of the liposomes not containing fragment A and fragment A. Similar results were obtained with Molt-4/HTLV-III cells treated with anti-HIV antibody. The culture medium of Molt-4/HTLV-III cells treated with anti-HIV antibody gave a somewhat low infectious virus titer, and this is probably due to the neutralization action of the antibody.

Table 1

| Treatment of Molt-4/HTLV-III with anti-HIV antibody | Specimen treated with * | HIV Infectivity ($\log_{10}$ $TCID_{50}$/ml) | |
|---|---|---|---|
| | | Medium | Cell |
| - | Lip(Fr.A) | $9.1 \times 10^4$ | $6.3 \times 10^4$ |
| - | Lip + Fr.A | $2.0 \times 10^6$ | $6.3 \times 10^6$ |
| - | Lip | $3.6 \times 10^5$ | $6.3 \times 10^6$ |
| - | Fr.A | $3.6 \times 10^5$ | $6.3 \times 10^6$ |
| - | PBS | $6.3 \times 10^5$ | $4.3 \times 10^6$ |
| + | Lip(Fr.A) | $1.2 \times 10^4$ | $2.0 \times 10^5$ |
| + | Lip + Fr.A | $6.3 \times 10^4$ | $4.3 \times 10^6$ |
| + | Lip | $4.3 \times 10^4$ | $9.1 \times 10^6$ |
| + | Fr.A | $3.6 \times 10^4$ | $6.3 \times 10^6$ |
| + | PBS | $6.3 \times 10^4$ | $6.3 \times 10^6$ |

*) Lip(Fr.A), liposomes containing fragment A; Lip + Fr.A, mixture of liposomes not containing fragment A (i.e. empty liposomes) and fragment A; Lip, liposomes not containing fragment A (i.e. empty liposomes); Fr.A, fragment A.

Claims

1. A therapeutic composition useful in the treatment of acquired immunodeficiency syndrome (AIDS) which comprises liposomes containing a toxin which specifically inhibits protein synthesis in cells.

EP 0 292 000 B1

2. A method of killing HIV-infected or HIV-producing cells in vitro which comprises contacting said cells with liposomes containing a toxin which specifically inhibits protein synthesis in cells.

3. The method according to claim 2, wherein the toxin is fragment A of diphtheria toxin.

4. A method for preparing a pharmaceutical composition for the treatment of acquired immunodeficiency syndrome (AIDS) which comprises encapsulating a toxin which specifically inhibits cellular protein synthesis into liposomes.

5. The method according to claim 4, wherein said toxin is fragment A of diphtheria toxin.

6. Use of a liposomal preparation containing a toxin which specifically inhibits cellular protein synthesis for the manufacture of a pharmaceutical composition, for the treatment of acquired immunodeficiency syndrome (AIDS).

7. The use according to claim 6, wherein said toxin is fragment A of diphtheria toxin.

**Patentansprüche**

1. Therapeutisches Mittel, geeignet für die Behandlung von erworbenem Immunschwächesyndrom (AIDS), umfassend Liposomen, die ein Toxin enthalten, welches spezifisch die Proteinsynthese in Zellen hemmt.

2. Verfahren zum Abtöten von HIV-infizierten oder HIV-produzierenden Zellen in vitro, umfassend das Inkontaktbringen dieser Zellen mit Liposomen, die ein Toxin enthalten, welches spezifisch die Proteinsynthese in Zellen hemmt.

3. Verfahren nach Anspruch 2, wobei das Toxin Fragment A des Diphtherietoxins ist.

4. Verfahren zur Herstellung eines Arzneimittels für die Behandlung von erworbenem Immunschwächesyndrom (AIDS), umfassend die Einkapselung eines Toxins, welches spezifisch die zelluläre Proteinsynthese hemmt, in Liposomen.

5. Verfahren nach Anspruch 4, wobei das Toxin Fragment A des Diphtherietoxins ist.

6. Verwendung eines Liposomen-Präparats, das ein Toxin enthält, welches spezifisch die zelluläre Proteinsynthese hemmt, zur Herstellung eines Arzneimittels für die Behandlung von erworbenem Immunschwächesyndrom (AIDS).

7. Verwendung nach Anspruch 6, wobei das Toxin Fragment A des Diphtherietoxins ist.

**Revendications**

1. Composition thérapeutique utilisable dans le traitement du syndrome immunodéficitaire acquis (SIDA), qui comprend des liposomes contenant une toxine qui inhibe spécifiquement la synthèse des protéines dans les cellules.

2. Procédé de destruction de cellules infectées par HIV ou productrices de HIV in vitro, qui comprend la mise en contact desdites cellules avec des liposomes contenant une toxine qui inhibe spécifiquement la synthèse des protéines dans les cellules.

3. Procédé selon la revendication 2, dans lequel la toxine est le fragment A de la toxine diphtérique.

4. Procédé de préparation d'une composition pharmaceutique pour le traitement du syndrome immunodéficitaire acquis (SIDA) qui comprend l'encapsulation dans des liposomes d'une toxine qui inhibe spécifiquement la synthèse cellulaire des protéines.

5. Procédé selon la revendication 4, dans lequel ladite toxine est le fragment A de la toxine diphtérique.

7

6. Utilisation d'une préparation liposomique contenant une toxine qui inhibe spécifiquement la synthèse cellulaire des protéines pour la préparation d'une composition pharmaceutique pour le traitement du syndrome immunodéficitaire acquis (SIDA).

7. Utilisation selon la revendication 6, dans laquelle ladite toxine est le fragment A de la toxine diphtérique.

Fig. 1

Molt-4/HTLV-Ⅲ
(a)

Molt-4
(b)

Number of viable cells (cell/ml)

Days of culture (day)

Fig. 2

Fig. 3

Fig. 4